# EUROPEAN PATENT APPLICATION

(11) **EP 1 357 117 A2**
(43) Date of publication of application: **29.10.2003**
(21) Application number: 03008171.5
(22) Date of filing: 08.04.2003
(51) Int. Cl.: C07D 295/108, G03F 7/031, C07C 229/34, C08F 2/50

(54) **Novel aminoketone derivatives and preparation process and uses of the same as photoinitiators**

(30) Priority: 26.04.2002 US 132165
(71) Applicant: Chiu, Chingfan Chris, Taipei Hsien (TW); Yang, Rhenda, Taipei Hsien (TW); Yang, Kung Shuo, Taipei Hsien (TW)
(72) Inventor: Chiu, Chingfan Chris, Taipei Hsien (TW); Yang, Rhenda, Taipei Hsien (TW); Yang, Kung Shuo, Taipei Hsien (TW)
(74) Representative: Hepworth, John Malcolm

(57) **Abstract**

Disclosed herein are novel morpholinoketone derivatives of formula (I): 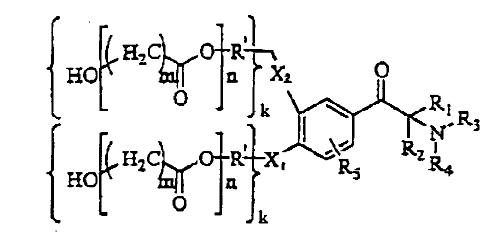 wherein each of the substituents is given the definition as set forth in the Specification and Claims.

Also disclosed are the preparation process of the derivatives, and their uses as a photoinitiator in photopolymerizable compositions.

## Description

### Cross-reference to related application

This application is a continuation-in-part of U.S. application Ser. No. 10/132,165, filed on April 26, 2002 and entitled "Novel morpholinoketone derivatives, and preparation process and uses of the same," the whole disclosure of which is incorporated herein by reference.

### Background of the invention

### 1) Field of the Invention

The present invention relates to a novel morpholinoketone derivative useful as a photoinitiator, and the preparation process and uses of the same.

### 2) Description of the Related Art

As compared to the conventional lithographic printing techniques, the UV curing printing technique has a broader spectrum of industrial applications, namely this technique can be applied not only to the manufacture of conventional printing inks such as the lithographic ink, the offset ink, the letterpress ink, the flexo ink, the gravure ink, the silk screen ink and the like, but also to the manufacture of printed circuit boards, photoresists, solder maskers and dielectric substrates, etc. In general, the UV curing process requires the use of a binder, a photopolymerizable monomer and a photoinitiator, in which the photoinitiator initiates the polymerization of the monomer upon the photoirradiation of UV light to thereby cause the production of a polymer in a short amount of time.

The photochemical polymerization of unsaturated monomers and prepolymers is a well known methodology and has wide industrial applications. U.S. Patent No. 3,661,614 discloses a radiation-curable solvent-free printing ink which consists essentially of: (1) about 20 to 98 weight percent of pentaerythritol acrylate, methacrylate or itaconate, (2) about 2 to 80 weight percent of a halogenated aromatic, alicyclic or aliphatic hydrocarbon photoinitiator, wherein the halogen atoms are attached directly to the ring structure in the aromatic and alicyclic compounds and to the carbon chain in the aliphatic compounds, and (3) a colorant.

U.S. Patent No. 4,672,079 discloses polymeric or polymerizable aromatic-aliphatic ketones, the preferred species of which are 2-hydroxy-2-methyl(4-vinylpropiophenone), 2-hydroxy-2-methyl-*p*-(1-methylvinyl)propiophenone, *p*-vinylbenzoylcyclohexanol, *p*-(1-methylvinyl)benzoyl-cyclohexanol and their oligomerization and polymerization products, they being suitable for use as photoinitiators for the photopolymerization of ethylenically unsaturated monomers and prepolymers.

U.S. Patent No. 4,943,516 discloses a photosensitive thermosetting resin composition which comprises (a) a photosensitive prepolymer containing at least two ethylenically unsaturated bonds in the molecular unit thereof, (b) a photoinitiator, (c) a photopolymerizable vinyl monomer and/or an organic solvent as a diluent, (d) a finely powdered epoxy compound containing at least two epoxy groups in the molecular unit thereof and exhibiting sparing solubility in the diluent to be used, and optionally (e) a curing agent for epoxy resin, and which excels in developing property and sensitivity and enjoys a long shelf life. When subjecting this photosensitive thermosetting resin composition to coating, exposure, development, and post curing, there can be formed a solder resist pattern which excels in adhesion, insulation resistance, and resistance to electrolytic corrosions, etc.

U.S. Patent No. 4,582,862 discloses photocurable colored compositions containing: (a) an olefinically unsaturated, photopolymerisable binder, (b) 5-60% weight of a pigment or a dye, and (c) 0.1-2% weight of a photoinitiator of formula (I) as disclosed therein. Specifically, this prior patent disclosed the following compound, *i.e.* 2-methyl-1-[4-(methylthio)phenyl]-2-morpholino-propane-1-one (MMMP), which has been widely used as a photoinitiator nowadays, in particular in the pigmented UV systems, such as a solder masker and a UV ink. It has be reported that MMMP can provide adequate photospeed performance and mechanical properties without sacrificing its non-yellowing property.

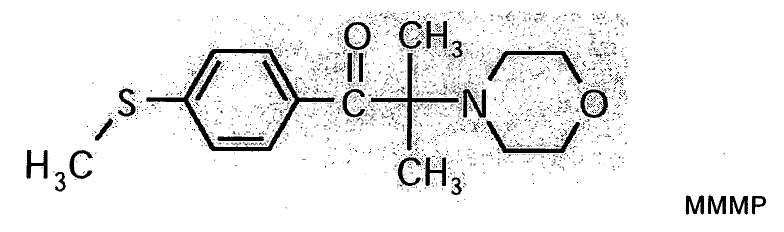

However, when MMMP is used, it will cause an odor problem after the post curing process due to the inherent volatile sulfur residue contained therein, thus limiting its industrial applications. In particular, when MMMP is employed in a UV ink or a solder masker, the problem of odor exists in varying degrees, ranging from mild to offensive. The odor is caused by the volatile chemical, i.e. 4-methylthiobenzaldehyde, which is produced from the photo-fragmentation of MMMP.

In view of the limited choices of photoinitiators for use, manufacturers normally have to tolerate the odor problem of MMMP, or use MMMP derivatives instead (see, e.g. *Luigi Angiolini et al., Journal of Applied. Polymer Science (1995), 55: 1477-1488;* US 4,582,862, US 5,145,885, US 5,506,279, US 5,837,746, WO 96/20795, and US 6,048,667), in particular the following compound, *i.e.* 2-benzyl-2-dimethylamino-1-(4- morpholinophenyl)-butan-1-one (BDMB) disclosed in US 5,145,885, which has been widely used as a replacement for MMMP.

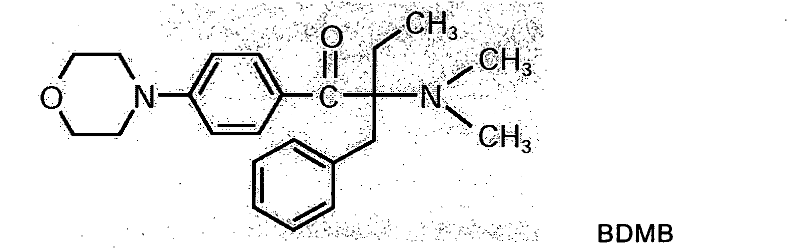

Furthermore, for some pigment systems using MMMP or BDMB as the major photoinitiator, such as the Cyan color, excess grinding is required for full incorporation of the photoinitiator. However, the grinding treatment oftentimes triggers undesirable premature curing and results in an increase in viscosity or even the gelling of inks. Therefore, the addition of a polymerization stabilizer such as MEHQ is required. However, the presence of this stabilizer will eventually retard the photo-speed. Moreover, MMMP and BDMB tend to increase the viscosity of an ink product incorporating the same, which is not favored for applications requiring low viscosity, such as the flexo inks.

Therefore, in view of the disadvantages present in the current technology as mentioned above, there exists a great need to develop a new material for use as a photoinitiator for photopolymerizable compositions.

### Summary of the invention

Accordingly, the present invention provides a compound of formula (I): 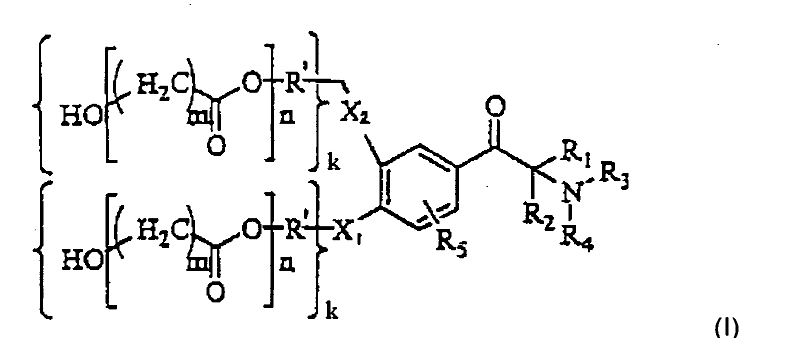 wherein
X₁ represents: H, S, N, O or NR where R is a C₁-C₁₂ alkyl group,
X₂ represents: H, S, N, O or NR where R is a C₁-C₁₂ alkyl group, with the proviso that when both X₁ and X₂ are not H, X₁=X₂;
R' represents: a C₁-C₁₂ alkylene group; or a group of the formula -(CH₂OCH₂)ₚ-, wherein p is an integer from 1 to 4;
R₁ and R₂ independently represent: H; phenyl; or C₁-C₁₂ alkyl, C₂-C₁₂ alkenyl or C₁-C₁₂ alkoxy optionally substituted by phenyl;
R₃ and R₄ independently represent a C₁-C₆ alkyl group optionally substituted by hydroxy;
or R₃ and R₄ together with the nitrogen atoms attached thereto form a cyclic group selected from a group consisting of 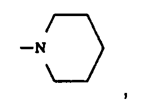 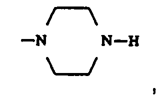 and 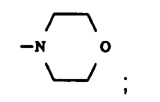 R₅ represents: H, F, Cl, Br, I, nitro, phenyl, C₁-C₁₂ alkyl or C₁-C₁₂ alkoxy;
when X₁ or X₂ is H, k is 0; when X₁ or X₂ is S, O or NR, k is 1;
when X₁ or X₂ is N, k is 2;
m is an integer from 2 to 5;
when k is 1, n is an integer from 1 to 20; and
when k is 2, n = n₁+n₂ ≤ 20, wherein each of n₁ and n₂ is an integer from 1 to 10.

The present invention also encompasses the applications of the compound of formula (I). For instance, the applicants have discovered that the compound of formula (I) is useful as a photoinitiator. Therefore, the compound of formula (I) according to the present invention can be formulated with a photopolymerizable monomer compound to form a photopolymerizable composition, such as a printing ink.

The above and other objects, features and advantages of the present invention will be apparent with reference to the following detailed description of the preferred examples.

### Detailed description of the invention

In order to overcome the odor problem of MMMP, the applicants have conducted many experiments to modify the molecular structure of MMMP by coupling a polymeric chain onto the methylthio moiety of MMMP, thus developing a compound of formula (I): 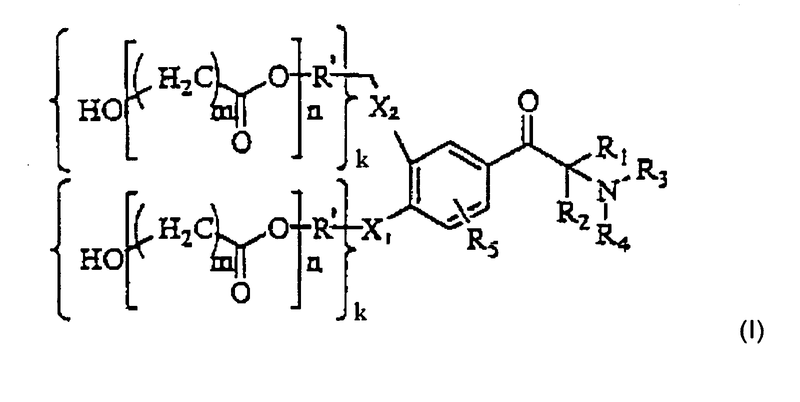 wherein
X₁ represents: H, S, N, O or NR where R is a C₁-C₁₂ alkyl group,
X₂ represents: H, S, N, O or NR where R is a C₁-C₁₂ alkyl group, with the proviso that when both X₁ and X₂ are not H, X₁=X₂;
R' represents: a C₁-C₁₂ alkylene group; or a group of the formula -(CH₂OCH₂)ₚ-, wherein p is an integer from 1 to 4;
R₁ and R₂ independently represent: H; phenyl; or C₁-C₁₂ alkyl,
C₂-C₁₂ alkenyl or C₁-C₁₂ alkoxy optionally substituted by phenyl;
R₃ and R₄ independently represent a C₁-C₆ alkyl group optionally substituted by hydroxy;
or R₃ and R₄ together with the nitrogen atoms attached thereto form a cyclic group selected from a group consisting of 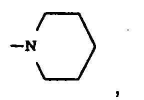 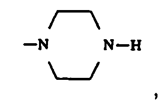 and 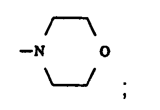 R₅ represents: H, F, Cl, Br, I, nitro, phenyl, C₁-C₁₂ alkyl or C₁-C₁₂ alkoxy;
when X₁ or X₂ is H, k is 0; when X₁ or X₂ is S, O or NR, k is 1;
when X₁ or X₂ is N, k is 2;
m is an integer from 2 to 5;
when k is 1, n is an integer from 1 to 20; and
when k is 2, n = n₁+n₂ ≤ 20, wherein each of n₁ and n₂ is an integer from 1 to 10.

In a preferred embodiment, m is 5.

In a preferred embodiment, R' is a C₁-C₆ alkylene group, in particular an ethylene group, or is a group represented by the formula -(CH₂OCH₂)- or -CH₂OCH₂CH₂OCH₂-.

Preferably, each of R₁ and R₂ is independently selected from a group consisting of methyl, ethyl, propyl, phenyl-substituted methyl, and phenyl-substituted propyl. In a preferred embodiment, R₁ and R₂ are both methyl. In another preferred embodiment, R₁ is phenyl-substituted methyl and R₂ is ethyl.

In a preferred embodiment, each of R₃ and R₄ is independently selected from a group consisting of methyl, ethyl, propyl, and hydroxy-substituted propyl. In a more preferred embodiment, R₃ and R₄ both are methyl.

In another preferred embodiment, R₃ and R₄ together with the nitrogen atoms attached thereto form a 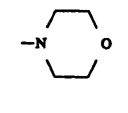 group.

In a preferred embodiment, R₅ is H, F, Cl, Br, I, phenyl, or C₁-C₆ alkyl. In a more preferred embodiment, R₅ is H.

In a preferred embodiment, one of X₁ and X₂ is S or O, and the other is H. In a more preferred embodiment, n is 3. In another preferred embodiment, n is 6.

In a more preferred embodiment, one of X₁ and X₂ is S, and the other is H; R' is an ethylene group; R₁ and R₂ independently represent methyl; R₃ and R₄ together with the nitrogen atoms attached thereto form a 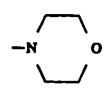 group; R₅ is H; and m is 5.

In another preferred embodiment, one of X₁ and X₂ is N, and the other is H; and the sum of n₁+ n₂ preferably is 6.

In a more preferred embodiment, one of X₁ and X₂ is N, and the other is H; R' is an ethylene group; R₁ and R₂ independently represent methyl; R₃ and R₄ together with the nitrogen atoms attached thereto form a 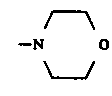 group; R₅ is H; m is 5; and n₁+ n₂ = 6.

In a further preferred embodiment, one of X₁ and X₂ is NR, (where R is a C₁-C₆ alkyl group, and the other is H. In a more preferred embodiment, one of X₁ and X₂ is NCH₃, and the other is H; R₁ is phenyl-substituted methyl and R₂ is ethyl; and both R₃ and R₄ are methyl. In another more preferred embodiment, one of X₁ and X₂ is NC₂H₅, and the other is H; R₁ is phenyl-substituted methyl and R₂ is ethyl; and both R₃ and R₄ are methyl.

The compound of formula (I) can be prepared by a process comprising the step of reacting a compound of formula (II) with a lactone compound of formula (III): 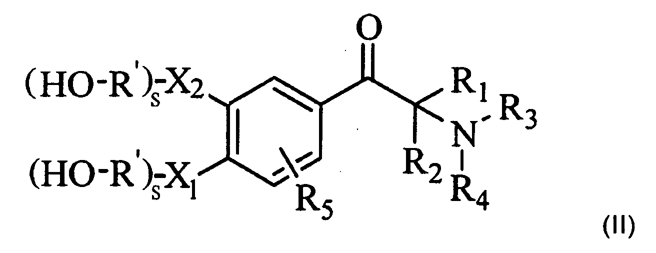 wherein
R₁, R₂, R₃, R₄, and R₅ are the same as those defined for formula (I) set forth above,
X₁ represents: H, S, N, O or NR where R is a C₁-C₁₂ alkyl group,
X₂ represents: H, S, N, O or NR where R is a C₁-C₁₂ alkyl group,
s is 0 or 1,
with the proviso that when both X₁ and X₂ are not H, X₁ = X₂, and
s is 1; and when X₁ or X₂ is H, s is 0; 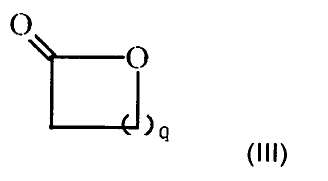 wherein q is an integer from 1 to 4.

Concerning the compound of formula (II), it can be prepared according to methodologies known in the art, e.g. the synthesis procedures disclosed in the prior art references cited above, including *Luigi Angiolini et al., Journal of Applied. Polymer Science (1995), 55: 1477-1488*; US 4,582,862, US 5,145,885, US 5,506,279, US 5,837,746, WO 96/20795 and US 6,048,667, etc.

The lactone compound of formula (III) suitable for use in the process according to the present invention may be selected from a group consisting of β-propiolactone, γ-butylolactone, δ-valerolactone and ε-caprolactone. In a preferred embodiment, the lactone compound of formula (III) is ε-caprolactone. These lactone compounds are commercially available, e.g. those available from the DAICEL Chemical Co.

It has been discovered that, by using the lactone compound of formula (III) as a source of the polymeric chain to couple onto a MMMP-like compound, the following advantages may be provided to the product derived therefrom:
1. the molecular weight of the product can be easily controlled;
2. high pigment wetting properties;
3. high UV stability;
4. low toxicity (biodegradable); and
5. low viscosity.

The process of the present invention can be conducted in the presence of a catalyst capable of initiating the ring-opening polymerization of the lactone compound of formula (III). The catalyst suitable for use in the process of the present invention includes, for instance, tetraethyl titanate, tetrapropyl titanate, tetrabutyl titanate or other titanate compounds, stannous octoate, dibutyl-tin-oxide, dibutyl-tin-dilaurate, n-butyl-tin tris(alkanoate) or other organic tin compounds, stannous chloride, stannous bromide, or other stannous halide compounds (see US 6,037,393).

In a preferred embodiment, the process of the present invention is conducted in the presence of a tin-containing catalyst. In a more preferred embodiment, the process of the present invention is conducted in the presence of n-butyl-tin tris(alkanoate), i.e. SCAT-24.

In a preferred embodiment, the compound of formula (II) used in the process according to the present invention is produced by reacting a compound of formula (II') with a compound of formula (IV): 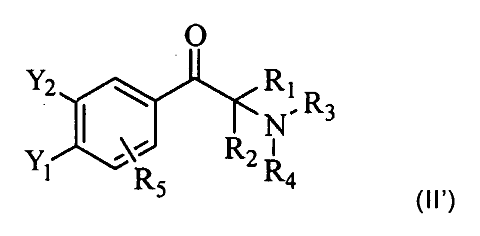 wherein
R₁, R₂, R₃, R₄, and R₅ are the same as those defined for formula (I) set forth above;
Y₁ represents: H, F, Cl, Br, I, or cyano,
Y₂ represents: H, F, Cl, Br, I, or cyano,
with the proviso that when both Y₁ and Y₂ are not H, Y₁ = Y₂;

**R"XR'OH** (IV)

wherein
X is S, NH, O or NR (where R is a C₁-C₁₂ alkyl group),
R' is the same as that defined for formula (I) set forth above, and when X is S, O or NR, R" is H; and when X is NH, R" is R'OH.

Regarding the compound of formula (II'), the preparation thereof can be done with reference to known methodologies disclosed in the prior art. For example, based on the relevant descriptions set forth in US 4,582,862, the compound of formula (II') may be prepared according to the following synthesis scheme, in which an amino-containing radical is introduced into a corresponding aryl alkyl ketone compound: 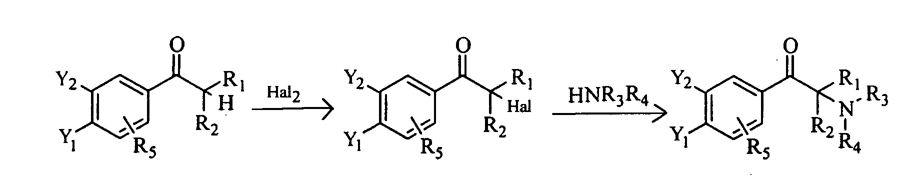 wherein Hal represents halogen, and R₁ to R₅ and Y₁ to Y₂ are the same as those defined for formula (II') described above.

In a preferred embodiment, in the compound of formula (II') used in the present invention, one of Y₁ and Y₂ is H and the other is F, Cl, Br or I. More preferably, one of Y₁ and Y₂ is H and the other is F or Cl. In another preferred embodiment, in the compound of formula (II') used in the present invention, Y₁ and Y₂ both are F or Cl.

Preferably, in the compound of formula (II') used in the present invention, each of R₁ and R₂ is independently selected from a group consisting of methyl, ethyl, propyl, phenyl-substituted methyl and phenyl-substituted propyl. In a preferred embodiment, both R₁ and R₂ are methyl. In another preferred embodiment, R₁ is phenyl-substituted methyl and R₂ is ethyl.

In a further preferred embodiment, in the compound of formula (II') used in the present invention, R₃ and R₄ together with the nitrogen atoms attached thereto form a 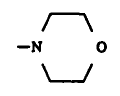 group.

Preferably, in the compound of formula (II') used in the present invention, each of R₃ and R₄ is independently selected from a group consisting of methyl, ethyl, propyl, and hydroxy-substituted propyl.

In another preferred embodiment, in the compound of formula (II') used in the present invention, R₅ is H, F, Cl, Br, I, phenyl, or C₁-C₆ alkyl. In a more preferred embodiment, R₅ is H.

The compound of formula (IV) is commercially available, e.g. available from BASF Corporation.

In a preferred embodiment, the compound of formula (IV) used in the present invention is HS(R'OH), where R' is an ethylene group. In another preferred embodiment, the compound of formula (IV) used in the present invention is NH(R'OH)₂, where R' is an ethylene group. In a further preferred embodiment, the compound of formula (IV) used in the present invention is RNH(R'OH), where R is a methyl or ethyl group and R' is an ethylene group.

In a preferred embodiment, to produce the compound of formula (I), the present invention employed a compound of formula (II') in which one of Y₁ and Y₂ is H, and the other is F, Cl, Br, or I; R₁ and R₂ independently represent methyl, R₃ and R₄ together with the nitrogen atoms attached thereto form a 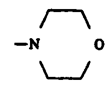 group; and R₅ is H; and a compound of formula (IV) which is HS(R'OH), where R' is an ethylene group. In addition, the thus formed compound of formula (II) is directly subjected to a reaction with ε-caprolactone, thereby producing the compound of formula (I), wherein one of X₁ and X₂ is S, and the other is H.

In another preferred embodiment, the present invention employed a compound of formula (II') in which one of Y₁ and Y₂ is H, and the other is F, Cl, Br, or I; R₁ and R₂ independently represent methyl; R₃ and R₄ together with the nitrogen atoms attached thereto form a 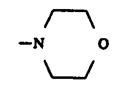 group; and R₅ is H; and a compound of formula (IV) which is NH(R'OH)₂, where R' is an ethylene group. The thus formed compound of formula (II) is then directly subjected to a reaction with ε-caprolactone to produce the compound of formula (I), where one of X₁ and X₂ is N, and the other is H; and n₁+ n₂ = 6.

In a further preferred embodiment, to produce the compound of formula (I), the present invention employed a compound of formula (II') in which one of Y₁ and Y₂ is H, and the other is F, Cl, Br, or I; R₁ and R₂ independently represent a group selected from methyl, ethyl, phenyl-substituted methyl, and phenyl-substituted ethyl; R₃ and R₄ independently represent a methyl group; and R₅ is H; and a compound of formula (IV) which is RNH(R'OH), where R is a methyl or ethyl group and R' is an ethylene group. In addition, the thus formed compound of formula (II) is directly subjected to a reaction with ε-caprolactone, thereby producing the compound of formula (I), wherein one of X₁ and X₂ is NR where R is methyl or ethyl, and the other is H.

The present invention also envisions the applications of the compound of formula (I). According to the present invention, the compound of formula (I) is useful as a photoinitiator, and it neither has the odor problem usually associated with MMMP nor undesirably increases the viscosity of a photopolymerizable composition containing the same. Therefore, according to the available methodologies that are well known in the art, the compound of formula (I) according to the present invention can be formulated with a photopolymerizable monomer, a photosensitizer, a pigment or a dyestuff, and other additives commonly employed by those skilled in the art in the preparation of a photopolymerizable composition, such as oligomers, photosensitizers, amine synergists and other physical property modifiers.

It is contemplated that the compound of formula (I) according to the present invention can be used in different industries, e.g. those involving the use of a photoinitiator as mentioned in the prior art references cited above. For example, the compound of formula (I) according to the present invention can be formulated in UV inks or coatings to act as a photoinitiator. It is also contemplated that the compound of formula (I) according to this invention can be used in combination with the conventional photoinitiators, such as those photoinitiators disclosed in the prior art references cited above, especially the ones disclosed in US 4,672,079.

The invention will now be described in more detail with reference to the following examples. However, it should be understood that these examples are given for the purpose of illustration only and are not intended to limit the scope of the present invention.

### <Example 1> Synthesis of MMMP-3 and MMMP-6

This example illustrates the synthesis of a compound of formula (I) according to this invention, e.g. 6-hydroxy-hexanoic acid 5-(5-{2-[4-(2-methyl-2-morpholin-4-yl-propionyl)-phenylsulfanyl]-ethoxycarbonyl}-pentyl-oxycarbonyl)pentyl ester (i.e. MMMP-3) and 6-hydroxy-hexanoic acid 5-(5-{2-[4-(2-methyl-2-morpholin-4-yl-propionyl)-phenylsulfanyl]-ethoxy carbonyl}-tetra(pentyloxycarbonyl)pentyl ester (i.e. MMMP-6), as well as their intermediate compound, i.e. 1-[4-(2-Hydroxyethylthio)-phenyl]-2-methyl-2-morpholinopropan-1-one (compound 2). The relevant synthesis scheme is shown below:

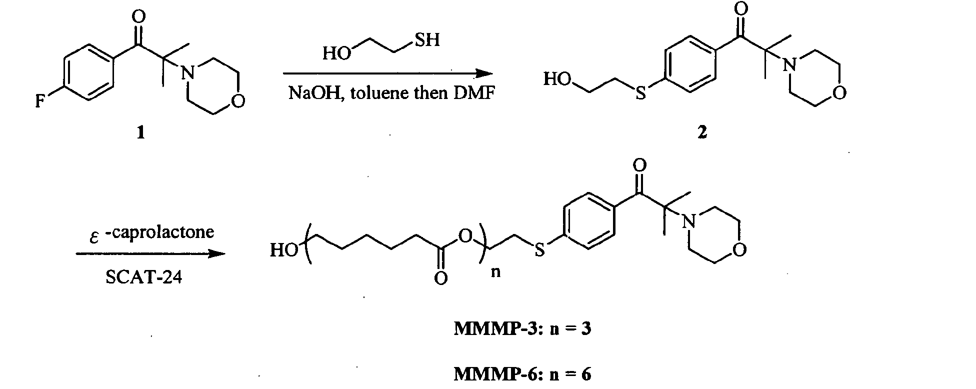

The molecular weight of the resultant compound may be varied by adjusting the molar ratio of compound 2 to ε-caprolactone, as evidenced by MMMP-3 and MMMP-6.

### A. Synthesis of 1-[4-(2-Hydroxyethylthio)-phenyl]-2-methyl-2-morpholino-propan-1-one (Compound 2)

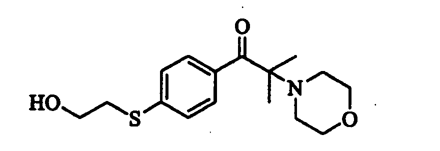

94.8 g (1.21 mol) of mercaptoethanol and 600 ml of toluene were placed into a 1 L three neck round-bottomed flask, and 48.6 g (1.21mol) of NaOH was further added. The mixture was brought to reflux for 6 hours while water was formed and collected by a Dean-Stark apparatus. The toluene solvent was subsequently removed by distillation, and 250 ml of DMF was added to the resultant solid cake. After the solution was cooled to room temperature, 250 g (0.93 mol) of 1-(4-Fluorphenyl)-2-methyl-2-morpholino-1-propanone (compound 1, prepared according to US 4,582,862) was added in one portion, and the resulting mixture was heated at 50°C overnight under the protection of nitrogen gas. The mixture was then cooled to room temperature and diluted with 1 L of toluene, followed by washing with water, 2% aq. NaOH solution, and brine in sequence. The organic layer was separated and concentrated *in vacuo* to give a yellow viscous oil, which was further purified by re-crystallization to produce an off-white crystal (90% yield, melting point: 62-64°C).

### B. Synthesis of MMMP-3

200g (0.65 mol) of compound 2, 88.5 g of ε-caprolactone, and 8 g of a tin catalyst (trade name SCAT-24, purchased from Sankyo Organic Chemistry Co., Ltd., Japan) were placed into a 500 ml three neck round-bottom flask equipped with a condenser and under the protection of nitrogen gas. The mixture was heated to a temperature of 80-85°C for 2 hours. 44.3 g of ε-caprolactone and 4 g of SCAT-24 were added into the above-mentioned flask every 40 minutes for 4 times, and the temperature was maintained for an additional hour. The resultant mixture was washed with water, 2% aq. NaOH solution, and brine in sequence. The organic layer was concentrated *in vacuo* to give a light tan oil (398 g, 95% yield).

### Detected properties of the title compound:

¹H NMR (CDCl₃, 400 MHz): δ 8.46 (d, *J* = 8.4 Hz, 2 H), 7.29 (d, *J* = 8.4 Hz, 2 H), 4.26 (t, *J* = 6.9 Hz, 2 H), 4.02 (t, *J* = 6.5 Hz, 6 H), 3.65 (t, *J* = 3.8 Hz, 4 H), 3.60 (t, *J* = 6.5 Hz, 3.20 (t, *J* = 6.9 Hz, 2 H), 2.53 (t, *J* = 3.8 Hz, 4 H), 2.25-2.30 (m, 9 H), 1.51-1.65 (m, 20 H), 1.30-1.42 (m, 10 H), 1.27 (s, 6 H). IR (KBr): 3501, 2942, 2865, 1741, 691, 1588, 1563, 1475, 1402, 1364, 1158, 1120, 1093, 980, 882, 761 cm⁻¹.

Based on the obtained ¹H NMR analysis, the n value for the compound product is calculated to be 3. As compared to the molecular weight of MMMP which is 279, the molecular weight of the compound product is 650.

### C. Synthesis of MMMP-6

MMMP-6 was synthesized by the same procedures for MMMP-3 with an exception that the amount of ε-caprolactone is 354 g instead of 88.5 g. The resultant product is a light tan paste (97% yield).

### Detected properties of the title compound:

¹H NMR (CDCl₃, 400 MHz): δ 8.46 (d, *J* = 8.4 Hz, 2 H), 7.29 (d, *J* = 8.4 Hz, 2 H), 4.26 (t, *J* = 6.9 Hz, 2 H), 4.02 (t, *J* = 6.5 Hz, 12 H), 3.65 (t, *J* = 3.8 Hz, 4 H), 3.60 (t, *J* = 6.5 Hz, 3.20 (t, *J* = 6.5 Hz, 2 H), 3.20 (t, J=6.9 Hz, 2 H), 2.53 (t, J=3,8 Hz, 4 H), 2.25-2.30 (m, 18 H), 1.51-1.65 (m, 40 H), 1.30-1.42 (m, 20 H), 1.27 (s, 6 H).

Based on the obtained ¹H NMR analysis, the n value for the compound product is calculated to be 6. As compared to the molecular weight of MMMP which is 279, the molecular weight of compound product is 960.

### D. Comparison of the physical properties of MMMP-3 and MMMP-6

MMMP-3 is a light amber color liquid at room temperature in comparison with MMMP-6 which is a paste. The congealing point of MMMP-3 is -15°C and it will not solidify at 0°C for 30 days. A comparison of the physical properties of MMMP-3 and MMMP-6 is summarized in the following Table 1.

**Table 1**

| | MMMP-3 | MMMP-6 |
|---|---|---|
| Color* (neat) | 4.5 | 4.8 |
| Viscosity at 70°F | 1200 cps | Paste |
| Viscosity at 100°F | 423 cps | 1743 cps |
| Density | 1.082 | 1.092 |

| | | |
|---|---|---|
| *: in Gardner unit | | |

### <Example 2>

This example illustrates the synthesis of another compound of formula (I) according to the present invention, namely compound 5, as well as its intermediate compound, *i*.*e*. (1-{4-[bis-(2-hydroxy- γ - ethyl)-amino]}-2-methyl-2-morpholino-1-propanone, compound 4). The relevant synthesis scheme is shown below:

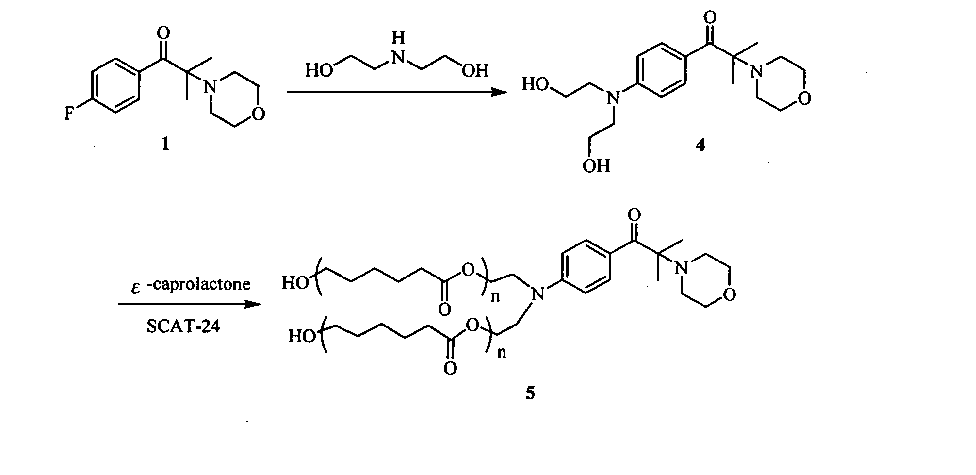

### A. Synthesis of 1-{4-[bis-(2-hydroxy- γ-ethyl)-amino]}-2-methyl-2-morpholino-1-propanone (compound 4)

10.0 g (0.037 mol) of 1-(4-Fluorphenyl)-2-methyl-2-morpholino-1-propanone and 58.9 g (0.56 mol) of diethanolamine were placed into a 100 ml three neck round-bottomed flask, and heated to a temperature 150°C for 24 hours. The resultant solution was cooled and diluted with 100 ml of EtOAc, followed by washing with water and brine. Removal of the solvent by a rotary evaporator gave a yellow oil product (11 g, 88% yield).

### B. Preparation of Compound 5

11.0 g (0.033 mol) of compound 4, 17.9 g of ε-caprolactone and 1.8 g of a tin catalyst (trade name SCAT-24, purchased from Sankyo Organic Chemistry Co., Ltd., Japan) were placed into a 100 ml three neck round-bottomed flask equipped with a condenser and under the protection of nitrogen gas. The resultant mixture was heated to a temperature of 80-85°C for 1.5 hours. A mixture of 9 g of ε-caprolactone and 0.9 g of SCAT-24 were added to the flask every 30 minutes for 3 times. The temperature was maintained for an additional hour. The reaction mixture was subsequently cooled to room temperature and diluted with 200 ml of toluene, followed by washing with water and brine. The organic layer was concentrated *in vacuo* to give a viscous oil product (29.8 g, 90% yield).

Based on the ¹H NMR analysis, the n₁+n₂ value for compound 5 was calculated to be 6.

### <Example 3>

This example illustrates the synthesis of a further compound of formula (I) according to the present invention, namely 2-oxepanone,2-{{4-[2-(dimethylamino)-1-oxo-2-(phenylmethyl)butyl]-phenyl}-methylamino}-ethyl ester (compound 3690), as well as its intermediate compound, namely 2-(Dimethylamino)-1-{4-[(2-hydroxyethyl)methylamino]phenyl}-2-phenylmethyl-1-butanone (compound 369N). The relevant synthesis scheme is shown below: 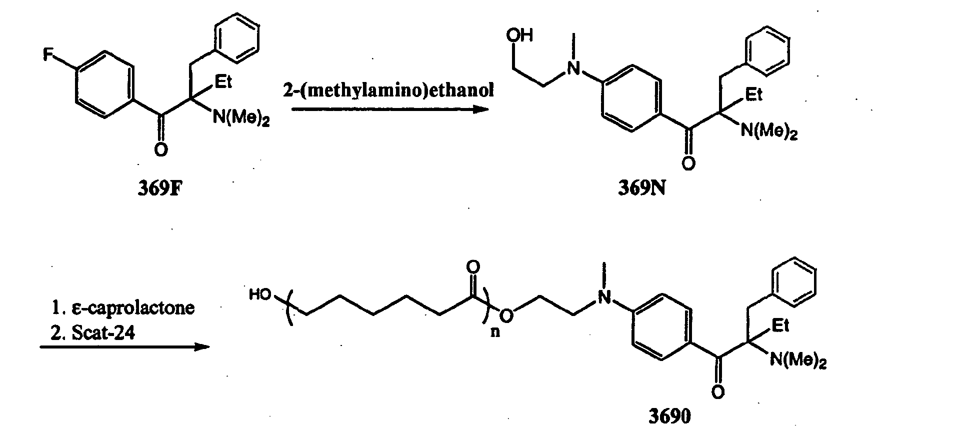 wherein n can be an integer of 1 to 10, preferably 1 to 6.

### A. Synthesis of 2-(Dimethylamino)-1-{4-[(2-hydroxyethyl)methylamino]-phenyl}-2-phenylmethyl-1-butanone (compound 369N)

100g of compound 369F (prepared according to the disclosure of US 5,077,402) and 600g of 2-(methylamino)ethanol were placed in a 2L three neck round-bottomed flask. The mixture was homogeneously stirred and then purged with N₂ for 5 times. After stirring the mixture at an elevated temperature of 150°C for 3 hrs, 50g of compound 369F was added into the flask, and the mixture was stirred for an additional 2 hrs. Thereafter, an additional 50 g of compound 369F was added into the flask and the mixture was stirred for an additional 4 hrs. After the mixture was cooled down to a temperature of 40°C, 470g of unreacted 2-(methylamino)ethanol was removed by distillation under the conditions of 48°C and 2.0 torr, and the resultant product was added with 200 g of toluene and 400 g of water, followed by homogeneous stirring. After removing the water layer, an additional 200 g of water was added, and the resultant mixture was stirred for 10 min. Compound 369N was obtained after removing the water layer and toluene in sequence.

### Detected properties of the title compound:

¹H NMR (CDCl₃, 200 MHz): δ 8.35 (d, *J* = 9.0 Hz, 2 H), 7.20-7.27 (m, 5 H), 6.66 (d, *J* = 9.0 Hz, 2 H), 3.82 (t, *J* = 6.0 Hz, 2 H), 3.56 (t, *J* = 6.0 Hz, 2 H), 3.22 (s, 2 H), 3.06 (s, 3 H), 2.38 (s, 6 H), 1.40-2.10 (m, 2 H), 0.72 (t, *J* = 7.4 Hz, 3 H).

### B. Synthesis of 2-oxepanone,2-{{4-[2-(dimethylamino)-1-oxo-2-(phenylmethyl)-butyl]-phenyl}-methylamino}-ethyl ester (compound 3690)

To the compound 369N obtained from the above step A, 150 g of ε-caprolactone was added. After being heated to a temperature of 150 °C, the mixture was added with 0.043 g of Scat-24. Four hours later, an additional 78 g of ε-caprolactone was added, and the resultant mixture was stirred for an additional 4 hrs. Finally, the unreacted ε - caprolactone was removed under the conditions of 50°C and 2.0 torr, to give a red-brown viscous liquid (401 g, 93.7% yield).

### Detected properties of the title compound (n =3):

¹H NMR (CDCl₃, 200 MHz): δ 8.36 (d, *J* = 8.7 Hz, 2 H), 7.19-7.27 (m, 5 H), 6.64 (d, *J* = 8.7 Hz, 2 H), 4.28 (t , 2 H), 4.03-4.06 (m, 4 H), 3.61-3.67 (m, 4 H), 3.19 (s, 2 H), 3.05 (s, 3 H), 2.60 (b, 1 H), 2.35 (s, 6 H), 2.27-2.31 (m, 6 H), 1.80-2.20 (m, 2 H), 1.61-1.69 (m, 12 H), 1.34-1.41 (m, 6 H), 0.68 (t, *J* = 7.4 Hz, 3 H).

IR (KBr): 3514, 2940, 2866, 1734, 1657, 1642, 1593, 1547, 1462, 1379, 1249, 1183, 823, 704, 662 cm⁻¹.

UV(λₘₐₓ) = 335.1 nm.

Based on the obtained ¹H NMR analysis, the obtained product is compound 3690 where n=3, and it has a molecular weight of around 650.

### <Experiment 1> Photospeed performance and odor property comparisons

This experiment is conducted to determine whether or not the compound of formula (I) according to the present invention is suitable for use as a photoinitiator as compared with the conventional commonly employed compound, MMMP. In this experiment, MMMP-3, MMMP-6 and compound 3690 according to this invention were respectively employed in the test inks having the compositions as shown in the following Table 2, and they were compared with MMMP in terms of the photospeed performance and the odor property.

### Materials:

1. MMMP: purchased from Ciba Specialty Chemicals Holding Inc., trade name Irgacure 907.
2. MMMP-3: a compound produced according to Example 1 set forth above.
3. MMMP-6: a compound produced according to Example 1 set forth above.
4. compound 3690: a compound produced according to Example 3 set forth above.
5. Ethyl Michler's Ketone: purchased from Chitec Chemical Co., Ltd., Taiwan, R.O.C., trade name Chivacure EMK, which is used as a photosensitizer in the photopolymerization of printing inks.
6. Isopropyl thioxanthone: purchased from Chitec Chemical Co., Ltd., Taiwan, R.O.C., trade name Chivacure ITX, which is used as a photosensitizer in the photopolymerization of printing inks.
7. Trimethylolpropane triacrylate, TMPA: purchased from UCB Chemical Co., which is used as a monomer compound in the photopolymerization of printing inks.
8. Eberyl 3702: purchased from UCB Chemical Co., which is used as an oligomer in the photopolymerization of printing inks.
9. Fastogen Blue 5380-E (C.I.B.-15.3): purchased from Dainippon Ink and Chemicals Co., which is used as a pigment in printing inks.

### Methods:

A. Compositions of the test inks: The above-mentioned materials are formulated into 3 different test inks according to the compositions shown in Table 2.

**Table 2.**

| Compositions of the test inks | | | | |
|---|---|---|---|---|
| | Ink 1 | Ink 2 | Ink 3 | Ink 4 |
| MMMP | 4*¹ | - | - | |
| MMMP-3 | - | 8*² | - | |
| MMMP-6 | - | - | 12 | |
| Compound 3690 | | | | 8*² |
| Ethyl Michler's Ketone | 0.5 | 0.5 | 0.5 | 0.5 |
| Isopropyl Thioxanthone | 1.0 | 1.0 | 1.0 | 1.0 |
| TMPTA | 25 | 25 | 25 | 25 |
| Ebecryl 3702 | 60 | 60 | 60 | 60 |
| Fastogen Blue 5380-E (C.I.B.-15:3) | 5 | 5 | 5 | 5 |
| Grinding with a three-roll miller | Twice | _*3 | - | - |

| | | | | |
|---|---|---|---|---|
| *¹: By weight | | | | |
| *²: Based on same equivalent weight. | | | | |
| *³: Blending is applied instead of grinding. | | | | |

B. Photopolymerization of the test inks: A 10 µm layer of a test ink was applied onto a hard board and cured to form a coating by a 300 W/in exposure using a Fusion D-type lamp (Fusion Model: F300S).
C. Test of the photospeed performance: The photospeed performance of the test ink was recorded in a unit of in/min until a tack-free coating formed.
D. Test of the Odor property: Coatings formed from the test inks were heated at a temperature of 80°C for a period of 5 minutes, and the odors emitted therefrom were assessed during this period of time.

The final assessment was categorized as "pungent" and "none."

### Results:

The four test inks listed in Table 2 were subjected to the photospeed performance and odor property tests, and the results thereof are shown in the following Table 3.

**Table 3**

| Comparison of photospeed performance and odor property. | | | | |
|---|---|---|---|---|
| | Ink 1 | Ink 2 | Ink 3 | Ink 4 |
| Photospeed (in/min) | 640 | 620 | 540 | 700 |
| Post-cure Odor | Pungent | None | None | None |

It can be clearly seen from Table 3 that the severe odor problem occurring in ink 1 does not appear in ink 2 (containing MMMP-3), ink 3 (containing MMMP-6) and ink 4 (containing compound 3690). Besides, the photospeed performance of ink 2 is similar to that of ink 1 based on the same equivalent weight. These results prove the effectiveness of the compounds of formula (I) according to this invention as a photoinitiator in the formulation of printing inks which can be cured to form a polymeric structure upon photoirradiation. Moreover, when a compound according to the present invention is employed, there exists an advantage in that during the preparation of a printing ink, the ingredients can be formulated directly by blending without the need for grinding.

### <Experiment 2> Viscosity performance comparison

This experiment was conducted to determine the influence of the compound of formula (I) according to this invention upon the viscosity performances of printing inks containing the same over a certain period of time, especially under transport and storage conditions during summer. In this experiment, MMMP-3, MMMP-6 and compound 3690 were compared with MMMP using the four test inks shown in Table 2, in which the test inks were subjected to a minimal heating treatment which mimics the transport and storage conditions of inks during summer.

### Method:

The test inks were placed in an oven set at a temperature of 60°C, and a portion of each of the test inks was removed and analyzed at certain intervals designated in the following Table 4. The viscosity was recorded in a unit of centipoise (CPS) by using a Brooksfield model RV DV-1 viscometer.

### Results:

The four test inks listed in Table 2 were tested in regard to their viscosity performances, and the obtained results are shown in Table 4.

**Table 4.**

| The viscosity performances of the test inks vs. time at 60°C. | | | | |
|---|---|---|---|---|
| Hours | Ink 1 | Ink 2 | Ink 3 | Ink 4 |
| 0 | 6280*¹ | 4400 | 4120 | 3800 |
| 24 | 6480 | 5000 | 4500 | gelled |
| 64 | 8270 | 6510 | 5700 | gelled |
| 200 | 12400 | 9640 | 9200 | gelled |

| | | | | |
|---|---|---|---|---|
| *¹: in CPS unit | | | | |

It is clear from Table 4 that the test inks, which contain MMMP-3, MMMP-6 and compound 3690, respectively, are shown to have initial viscosities lower than that of the test ink containing the conventional MMMP compound. In addition, the test inks, which contain MMMP-3 and MMMP-6, respectively, are more resistant to gelation.

### <Experiment 3> Comparison of MMMP-3 and compound 3690 with BDMB

In this experiment, MMMP-3 and compound 3690 are further compared with BDMB in terms of the photospeed performance and the in-can stability in the absence of any stabilizer.

### Materials:

1. BDMB: Available from Ciba Specialty Chemicals Holding Inc., trade name Irgacure 369.
2. MMMP-3: A compound prepared according to Example 1 set forth above.
3. compound 3690: A compound prepared according to Example 3 set forth above.
4. Ethyl MiChler's ketone: see Experiment 1 described above.
5. Isopropyl thioxanthone: see Experiment 1 described above.
6. TMPTA: see Experiment 1 described above.
7. Eberyl 3702: see Experiment 1 described above.
8. Carbon Black: Available from Degusa, which is used as a pigment in printing inks.

### Methods:

A. Compositions of test inks: The above-mentioned materials are formulated into three different test inks according to the compositions shown in Table 5, with or without a grinding treatment using a standard 3-roller mill.

**Table 5.**

| Compositions of the test inks | | | |
|---|---|---|---|
| | Ink 5 | Ink 6 | Ink 7 |
| BDMD | 5 | - | - |
| MMMP-3 | - | 10 | - |
| Compound 3690 | - | - | 10 0 |
| Ethyl MiChler's ketone | 0.5 | 0.5 | 0.5 |
| Isopropyl thioxanthone | 1.0 | 1.0 | 1.0 |
| TMPTA | 25 | 25 | 25 |
| Ebecryl 3702 | 60 | 60 | 60 |
| Carbon black | 3 | 3 | 3 |
| Times for grinding | 3 times | - | - |

B. Photopolymerization of the test inks: Conducted according to the method of Experiment 1.
C. Test of photospeed: Conducted according to the method of Experiment 1.
D. Test of odor: Conducted according to the method of Experiment 1.
E. Test of viscosity performance: Conducted according to the method of Experiment 2.

### Results:

The three test inks listed in Table 5 were subjected to the photospeed performance, odor property and viscosity performance tests, and the results thereof are shown in the following Table 6.

**Table 6:**

| Comparison of MMMP-3 and compound 3690 with BDMB | | | |
|---|---|---|---|
| | Ink 5 | Ink 6 | Ink 7 |
| Photospeed (in/min) | 550 | 530 | 560 |
| Post-cure odor | None | None | None |
| Viscosity(cps, initial) | 6670 | 3740 | 4600 |
| Viscosity (24 hours at 60°C) | gelled | 3780 | gelled |
| Viscosity (100 hours at 60°C) | gelled | 6200 | gelled |

### Conclusion:

It is clear from the above-illustrated experiments and description that, when compared to the conventional commonly employed MMMP and BDMB compounds, the MMMP-3 and MMMP-6 compounds and compound 3690 according to the present invention, in particular MMMP-3, have unique characteristics as follows:
1. The Odor problem is completely overcome.
2. The photospeed performance of MMMP-3 according to this invention is comparable to those of the conventional MMMP and BDMB compounds based on the same equivalent weight.
3. Much lower viscosity can be achieved, which is favored by flexo and gravure ink applications.
4. The problem of pre-mature gelling no longer occurs, and the maintenance of constant viscosity and a longer shelf life for the formula composition are achievable.
5. The use of stabilizers may not be necessary, which not only simplifies the formulation procedure but also reduces the cost of manufacture.
6. No grinding treatment is needed, thus reducing the labor cost and time.

In addition, the drying speed of compound 3690 is faster than that of MMMP-3 and comparable to the conventional BDMB compound. While compound 3690, like the conventional BDMB compound, may bring about the problem of gelation, this problem can be resolved by addition of 0.5% stabilizer. Besides, when compound 3690 according to this invention is used, no grinding will be needed and the viscosity of ink system can be lowered. Accordingly, compound 3690 according to this invention is superior to the conventional BDMB compound.

All patents and literature references cited in the present specification are hereby incorporated by reference in their entirety. In case of conflict, the present description, including definitions, will prevail.

While the invention has been described with reference to the above specific embodiments, it is apparent that numerous modifications and variations can be made without departing from the scope and spirit of this invention. It is therefore intended that this invention be limited only as indicated by the appended claims.

## Claims

1. A compound of the formula (I) : 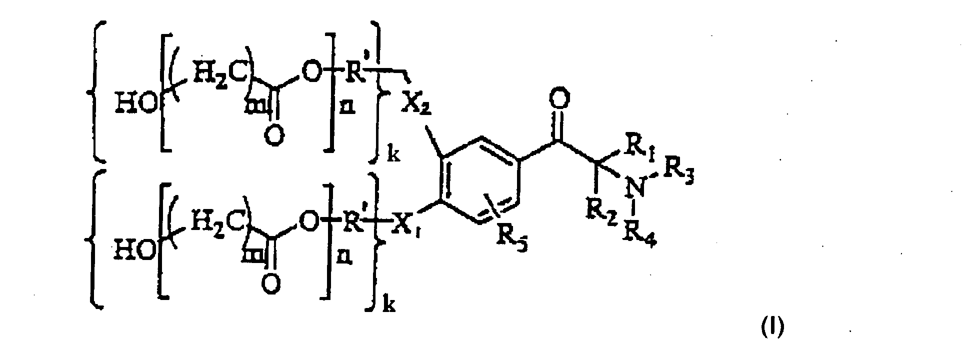 wherein
X₁ represents: H, S, N, O or NR where R is a C₁-C₁₂ alkyl group,
X₂ represents: H, S, N, O or NR where R is a C₁-C₁₂ alkyl group, with the proviso that when both X₁ and X₂ are not H, X₁ = X₂;
R' represents: a C₁-C₁₂ alkylene group; or a group of the formula -(CH₂OCH₂)ₚ-, wherein p is an integer from 1 to 4;
R₁ and R₂ independently represent: H; phenyl; or C₁-C₁₂ alkyl, C₂-C₁₂ alkenyl or C₁-C₁₂ alkoxy optionally substituted by phenyl;
R₃ and R₄ independently represent a C₁-C₆ alkyl group optionally substituted by hydroxy;
or R₃ and R₄ together with the nitrogen atoms attached thereto form a cyclic group selected from a group consisting of 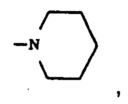 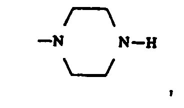 and 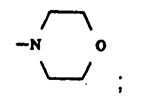 R₅ represents: H, F, Cl, Br, I, nitro, phenyl, C₁-C₁₂ alkyl or C₁-C₁₂ alkoxy;
when X₁ or X₂ is H, k is 0; when X₁ or X₂ is S, O or NR, k is 1;
when X₁ or X₂ is N, k is 2;
m is an integer from 2 to 5;
when k is 1, n is an integer from 1 to 20; and
when k is 2, n = n₁+n₂ ≤ 20, wherein each of n₁ and n₂ is an integer from 1 to 10.

2. A compound as claimed in claim 1, wherein R' is an ethylene group.

3. A compound as claimed in claim 1 or claim 2, wherein one of X₁ and X₂ is S or O, and the other is H.

4. A compound as claimed in any of the preceding claims, wherein m is 5.

5. A compound as claimed in claim 4, wherein n is 3.

6. A compound as claimed in any of the preceding claims, wherein each of R₁ and R₂ is independently selected from a group consisting of methyl, ethyl, propyl, phenyl-substituted methyl and phenyl-substituted propyl.

7. A compound as claimed in claim 6, wherein both R₁ and R₂ are methyl.

8. A compound as claimed in claim 6, wherein both R₁ is phenyl-substituted methyl and R₂ is ethyl.

9. A compound as claimed in any of the preceding claims, wherein each of R₃ and R₄ is independently selected from a group consisting of methyl, ethyl, propyl, and hydroxyl-substituted propyl.

10. A compound as claimed in any of the preceding claims, wherein R₃ and R₄ together with the nitrogen atoms attached thereto form a 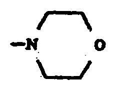 group.

11. A compound as claimed in any of the preceding claims, wherein:
one of X₁ and X₂ is S, the other is H,
R' is an ethylene group,
R₁ and R₂ independently represent methyl,
R₃ and R₄ together with the nitrogen atoms attached thereto form a 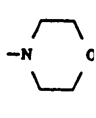 group,
R₅ is H, and
m is 5.

12. A compound as claimed in claim 11, wherein n is 3.

13. A compound as claimed in any of the preceding claims, wherein one of X₁ and X₂ is N, and the other is H.

14. A compound as claimed in any of the preceding claims, wherein:
one of X₁ and X₂ is N, and the other is H,
R' is an ethylene group,
R₁ and R₂ independently represent methyl,
R₃ and R₄ together with the nitrogen atoms attached thereto form a 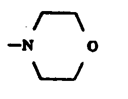 group,
R₅ is H,
m is 5, and
n₁+n₂=6.

15. A compound as claimed in any of the preceding claims, wherein:
one of X₁ and X₂ is NR, where R is methyl or ethyl, and the other is H,
R' is an ethylene group,
R₁ is phenyl-substituted methyl,
R₂ is ethyl,
R₃ and R₄ independently represent methyl,
R₅ is H, and
m is 5.

16. A photoinitiator comprising a compound as claimed in any of the preceding claims.

17. A photopolymerizable composition comprising a photopolymerizable monomer compound and a compound of formula (I) as claimed in any of claims 1 to 15.

18. A photopolymerizable composition as claimed in claim 17, further comprising a pigment of dyestuff.
